# EUROPÄISCHE PATENTSCHRIFT

(11) **EP 1 077 765 B1**
(45) Veröffentlichungstag und Bekanntmachung des Hinweises auf die Patenterteilung: **25.06.2003**
(21) Anmeldenummer: 99920696.4
(22) Anmeldetag: 17.04.1999
(51) Int. Cl.: B01J 19/12, C07C 29/50, C07C 45/32, C07D 309/04, C07J 75/00

(54) **SOLAR-PHOTO- UND SOLAR-THERMOCHEMISCHE SYNTHESEN MITTELS FLACHBETT-SONNENLICHTSAMMLER/-SOLARREAKTOREN**
PHOTOCHEMICAL AND THERMOCHEMICAL SOLAR SYNTHESES USING FLAT-BED SOLAR COLLECTORS/SOLAR REACTORS
SYNTHESES PHOTOCHIMIQUES SOLAIRES ET THERMOCHIMIQUES SOLAIRES EFFECTUEES A L'AIDE DE COLLECTEURS DE LUMIERE SOLAIRE/REACTEURS SOLAIRES PLATS

(30) Priorität: 17.04.1998 DE 19816876; 25.09.1998 DE 19844037
(43) Veröffentlichungstag der Anmeldung: 28.02.2001
(73) Patentinhaber: Studiengesellschaft Kohle mbH, 45470 Mülheim (DE)
(72) Erfinder: DEMUTH, Martin, D-45470 Mülheim an der Ruhr (DE); RITTER, Alfred, D-45470 Mülheim an der Ruhr (DE)
(74) Vertreter: von Kreisler, Alek, Dipl.-Chem.
(86) Internationale Anmeldenummer: EP9902597
(87) Internationale Veröffentlichungsnummer: WO99054032

(56) Entgegenhaltungen:
- EP-A- 0 761 808
- DE-A- 2 712 411
- DE-A- 4 344 163
- DE-C- 3 727 630
- US-A- 4 004 573
- US-A- 4 609 444
- US-A- 5 543 016
- PATENT ABSTRACTS OF JAPAN vol. 8, no. 77 (C-218), 10. April 1984 (1984-04-10) -& JP 59 004436 A (TOKYO SHIBAURA DENKI K.K.), 11. Januar 1984 (1984-01-11) -& DATABASE WPI Section Ch, Week 8408 Derwent Publications Ltd., London, GB; Class J04, AN 84-044993 XP002113790 & JP 59 004436 A (TOKYO SHIBAURA DENKI K.K.)
- PATENT ABSTRACTS OF JAPAN vol. 14, no. 198 (M-0965), 23. April 1990 (1990-04-23) -& JP 02 040453 A (MATSUSHITA ELECTRIC IND. CO.), 9. Februar 1990 (1990-02-09)
- DE LA ROSA M.A. ET AL.: 'Stabilization by high pH of hydrogen peroxide production with flavin photosystem' PHOTOBIOCHEMISTRY AND PHOTOPHYSICS Bd. 5

## Beschreibung

Die Erfindung betrifft ein Verfahren zur Durchführung solar-photo- und solarthermochemischer Synthesen mit Hilfe eines Flachbett-Sonnenlichtsammlers, welcher keine Vorrichtung zur Fokussierung oder Konzentrierung des Lichts benötigt und nicht dem Lauf der Sonne folgt.

Zur Durchführung solar-photochemischer Reaktionen sind gemäß DE 4134614 C2 mehrere solartechnische Vorrichtungen offenbart worden, bei welchen die solare Strahlung nicht direkt in das Reaktionsmedium gelangt, sondern erst nach dem Passieren vorgeschalteter Hilfsvorrichtungen zu deren Konzentrierung. Da die von der Sonne ausgesandte solare Strahlung orts- und wetterabhängig in unterschiedlichem Umfang diffus gestreut wird und somit nur noch zum Teil zur Strahlungskonzentration genutzt werden kann, lassen strahlungskonzentrierende Solarsysteme einen erheblichen Anteil des solaren Angebotes fiir photochemische Reaktionen ungenutzt. In DE 43 44163 A1 wird hervorgehoben, dass man bei der Photooxidation von Terpenolefinen weniger Nebenprodukte bei höheren Umsätzen erhält, sofern bei hoher Bestrahlungsdichte, gearbeitet wird.

DE 37 27 630 C offenbart ein Verfahren zur Durchführung eine photochemische Oxidation von H₂O zu H₂O₂ zur Speicherung von Solarenergie, wobei die Reaktionen in einem Flachbett-Sonnenlichtsammler bei Einstrahlung von Sonnenlicht durchgeführt werden.

Andererseits ist in unseren geografischen Breiten mangels direkter Sonneneinstrahlung die Anwendung von konzentrierenden oder fokussierenden Vorrichtungen für (photo)chemische Umsetzungen von Nachteil.

Es wurde nun überraschenderweise gefunden, daß sich dieser gravierende Nachteil in der Nutzung solarer Strahlungsenergie für photo- und thermochemische Zwecke vermeiden läßt durch den Einsatz eines technisch viel einfacheren und damit erheblich billigeren Flachbett-Sonnenlichtsammlers, weil letzterer auch diffuse Strahlung sowie Kunstlicht nutzen kann, ohne daß sich wesentliche Unterschiede in der Produktverteilung bei den so durchgeführten Reaktionen zeigen. Darüber hinaus erlaubt die Kombination von Sonnenlicht und Kunstlicht im Nachtbetrieb eine kontinuierliche Produktion. Desweiteren stellte sich durch vergleichende Experimente heraus, dass die Behauptung von Scharf (*Angew. Chem. Int. Ed. Engl. 33,* 2009, (1994), dass Konzentrierung des Sonnenlichts zu höheren Raum-Zeit-Ausbeuten führt, falsch und irreführend ist. In unseren geographischen Breiten sind die Raum-Zeit-Ausbeuten sogar höher als bei Benutzung von Konzentratoren. Das wesentliche Einsatzgebiet von Flachbett-Sonnenlichtsammlem war bisher die Detoxifizierung, insbesondere von Luft und Wasser, d. h. Abbau von niedrigmolekularen Stoffen.
Wir haben gefunden, dass sich Flachbett-Sonnenlichtsammler für die Synthese (Aufbau) von stereochemisch und strukturell komplexeren und höher molekularen Stoffen besonders gut eignen.

Zur Erzielung zufriedenstellender Produktausbeuten ist zudem eine Nachführung nach dem Sonnenstand kein Erfordernis für den Einsatz des Flachbett-Sonnenlichtsammler, was technisch erheblich einfacher ist und sich damit preismindernd auf die Anlagekosten auswirkt.

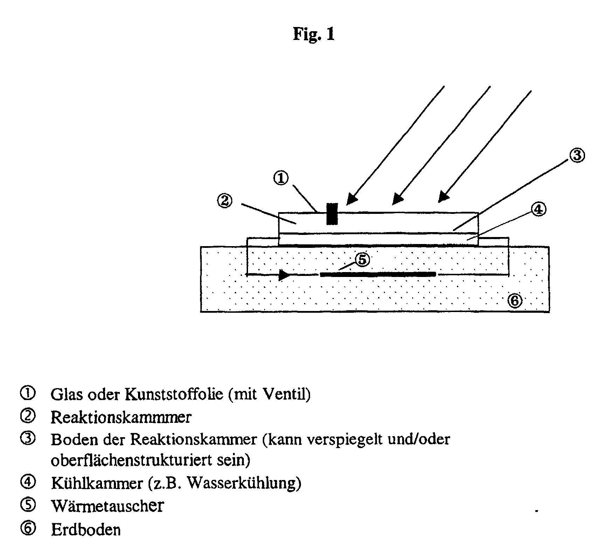

Als in hohem Maße ausbeutefördernd hat sich das Vorhandensein einer verspiegelten Fläche - zweckmäßigerweise in Form einer oberflächenstrukturierten strahlungsreflektierenden Metall- oder metallbedampften Kunstststoff-Folie - auf den Innenseiten des Flachbett-Sonnenlichtsammlers, z. B. auf dem Reaktorboden erwiesen (Fig. 1). Hierdurch bleibt solare Strahlung, welche beim Durchgang durch das Reaktionsmedium in Richtung Reaktorboden nicht absorbiert wird und somit photochemisch nicht reagiert, durch Reflexion für die weitere Nutzung verfügbar. Um den Wellenlängenbreich der einfallenden Solarstrahlung den jeweiligen Anforderungen anpassen zu können, wird der Reaktor oberseitig mit einem transparenten, UV-durchlässigen Glas oder vorzugsweise Kunststoff-Folie flüssigkeitsdicht verschlossen. Bei der Durchführung photochemischer Reaktionen, welche unter Bestrahlung mit z. B. UV-Licht oder anderen Wellenlängenbereichen schlecht ablaufen, kann durch Abdecken der vorstehend erwähnten Folie z. B. mit einer UV-undurchlässigen Schicht der UV-Anteil oder andere Wellenlängenbereiche im Strahlungsangebot ausgeblendet werden.

In einer besonderen Ausführungsform gemäß Fig. 1 kann der Flachbett-Sonnenlichtsammler zwecks Durchführung gekoppelter oder konsekutiv ablaufender photonischer und thermischer Prozesse mit einem Rohrwendel-Wärmetauscher versehen sein, welcher sich in einer separaten, mit Thermoöl oder einem anderen Wärmeträger gefüllten Kammer (Kühlkammer) unterhalb des Reaktorbodens befindet. Von dort aus erfolgt die Wärmeübertragung in die Reaktionskammer durch Wärmeleitung. Die thermische Energie für die Erwärmung des Reaktionsmediums wird zweckmäßigerweise von einem Solar-Kollektor oder Flachbett-Sonnenlichtsammler geliefert, welcher über eine temperaturgesteuerte Förderpumpe dem Rohrwendel-Wärmetauscher Prozesswärme zuführt.

Zwecks Durchführung photochemischer Reaktionen, welche im Niedertemperaturbereich bei ca. +10 °C ablaufen müssen, kann der Kollektor gemäß Fig. 1 durch einen Wärmetauscher, vorzugsweise einen Erdreichwärmetauscher - welcher sich in etwa 1 m Tiefe oder tiefer befinden sollte - erweitert werden (Fig. 1). Hierdurch ist der Reaktor unabhängig von Kühlung durch Stadtwasser oder maschinell erzeugter Kälte.

In einer besonderen Ausführungsform können mehrere Flachbett-Sonnenlichtsammler in einem Durchflußsystem miteinander verbunden werden. Das Syntheseprodukt wird dann dem letzten der in Reihe geschalteten Kollektoren entnommen. Ein geeigneter Detektor (z. B. auf der Basis von Lichtabsorption) sorgt für die Durchflußsteuerung mittels einer Pumpe.

Es können mit dem Flachbett-Sonnenlichtsammler beispielsweise Elektronenübertragungsreaktionen zur Synthese pharmazeutisch interessanter Zwischenprodukte durchgeführt werden (Beispiele 1-5) (fiir Anwendungen solcher Synthesen, siehe *Helv. Chim. Acta* 78, 2065 (1995) und *J*. *Am. Chem. Soc. 119,* 1129 (1997)). Es können insbesondere mit dem Flachbett-Sonnenlichtsammler auf einfachste Weise Steroide und Derivate davon hergestellt werden (siehe Beispiele 6 und 7). Solche werden industriell von z.B. Schering AG in grossen Mengen hergestellt; diese Produkte sind für die Hormonregulierung im Handel anerkannt (siehe Beispiel 6).

Weiterhin eignet sich der Einsatz des Flachbett-Sonnenlichtsammlers für die Photooxidation (Photooxygenierung) von Terpenolefinen und damit zur Synthese von industriell interessanten Geruchsstoffen, wie dem Rosenoxid, Myrtenol und Myrtenal (Parfum-Industrie; bisherige industrielle Herstellung mittels konventioneller Lampentechnik z.B. durch Dragoco, Givaudan, Firmenich und Reimer & Haarmann). Der Flachbett-Sonnenlichtkollektor eignet sich z.B. zur Herstellung des Duftstoffes Rosenoxid (siehe Beispiele 8 und 9).

Es können aber auch photochemische Anlagerungsreaktionen (Beispiele 1-7, 10 und 12) und Umlagerungen (Beispiele 10 und 11) mit diesem Sonnenlichtsammler durchgeführt werden (für weitereAnwendungen dieser Photochemie, siehe *Synthesis 1989*, 145).

### Beispiele:

Vergleich Parabolrinnenkonzentrator / Flachbett-Sonnenlichtsammler (am Beispiel der solar-photochemischen Synthese von (B) mittels Elektronenübertragung)

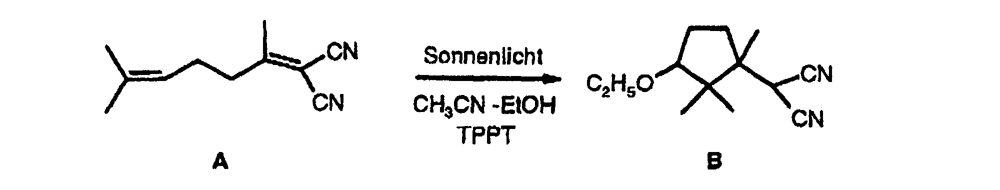

Die angegebenen Ausbeuten beziehen sich auf Mengen isolierten Produktes. Um die Versuche der Beispiele 1-3 vergleichen zu können, wurde jeweils alle 30 Minuten eine Probe gezogen und der Umsatz gemessen. Bei Umsätzen von etwa 50% wurden die Versuche abgebrochen und die Reaktionszeiten verglichen (siehe unten). Der Umsatz steigt in etwa bis zu 50% Umsatz linear mit der Zeit an. Die hier aufgeführten Beispiele/Experimente wurden in der Solaranlage auf dem Dach des MPI für Strahlenchemie in Mülheim an der Ruhr durchgeführt.

### Beispiel 1

Eingesetzte Lösung:
5,8 g ≃ 33,33 mMol 2,6-Dimethylhepta-1,5-diene-1,1-dicarbonitril (A),
5,3g ≃ 13,84 mMol 2,4,6 Triphenylpyrylium-tetrafluorobora (TPPT),
800 ml Acetonitril und 30 ml Ethanol.
Diese Lösung wurde mit Argon entgast und auch während der Belichtung unter Schutzgas gehalten.

600 ml dieser Lösung wurden im Parabolrinnenkonzentrator insgesamt 8 Stunden belichtet. Hiervon waren ca. 2 Stunden bewölkt, die restlichen Stunden waren Sonnenschein: Ausbeute 2.7 g; 51% (**B**).

### Beispiel 2

230 ml der Lösung von Beispiel 1 wurden im Flachbett-Sonnenlichtsammler belichtet. Nach 4 Stunden wurde kein Eddukt mehr nachgewiesen. In der Belichtungszeit von 4 Stunden waren ca. 2 Stunden mit Bewölkung: Ausbeute 0,98 g; 48% (**B**).

### Beispiel 3

230 ml der Lösung von Beispiel 1 wurden im Flachbett-Sonnenlichtsammler 6 Stunden bei bedecktem Himmel ohne direkte Sonnenstrahlung belichtet: Ausbeute 1 g; 49% (**B**).

Analytische Daten zur Struktursicherung von A und B, siehe laufende Doktorarbeit von K. D. Warzecha, MPI für Strahlenchemie/ Universität Essen.

Ergebnis: Die Reaktionszeit bis zum Erreichen einer Ausbeute von ca. 50 % ist mit vier Stunden im Beispiel 2 bzw. sechs Stunden im Beispiel 3 bei Benutzung eines Flachbett-Sonnenlichtsammlers geringer als im Beispiel 1 mit Parabolrinnenkonzentrator mit acht Stunden Reaktionszeit trotz sechs Stunden Sonnenschein. Vergleichende Experimente mit anderem Lösungsmittelgemisch:

### Beispiel 4

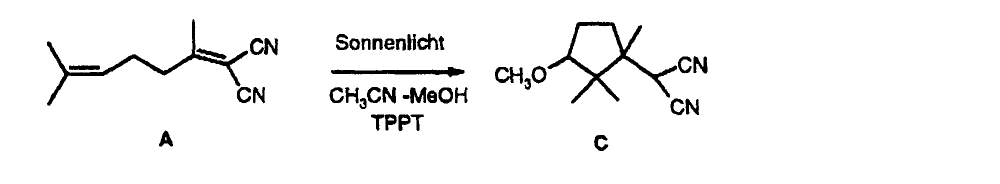

Eingesetzte Lösung:
3,4 g ≃ 19 mMol 2,b-Dimethylhepta-1,5-diene-1,1-dicarbonitril (B)
5 g ≃ 12,62 mMol 2,4,6 Triphenylpyrylium-tetrafluoroborat (TPPT)
1275 ml Acetonitril und 125 ml Methanol
700ml dieser Lösung wurden im Flachbett-Sonnenlichtsammler 3 Stunden belichtet: Erste Stunde teils sonnig, teils bewölkt; zweite Stunde bewölkt teilweise Regen; dritte Stunde bewölkt, teilweise Regen.
Ausbeute ca. 1,35 g; 63% (C)

### Beispiel 5.

Parallel zu Beispiel 4 wurden unter identischen Lichtverhältnissen 700 ml der Lösung von Beispiel 4 in einem Parabolrinnenkonzentrator belichtet: Weniger als 10% Produktausbeute.

Folgerung: Beispiele 4 und 5 zeigen, daß bei überwiegender Bewölkung der Flachbett-Sonnenlichtsammler der Parabolrinne erheblich überlegen ist.

Analytische Daten zur Struktursicherung von A und C, siehe laufende Doktorarbeit von K. D. Warzecha, MPI für Strahlenchemie/ Universität Essen.

### Verwendungsbeispiele:

### Beispiel 6

Steroidsynthese (komplexes Gerüst) 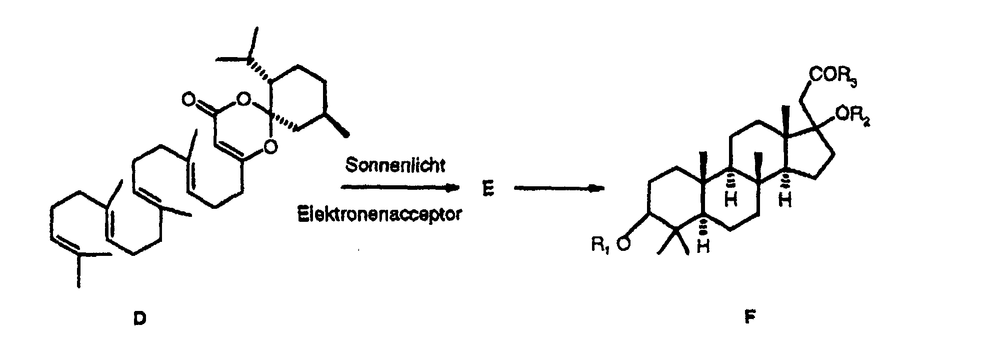
**D** (1,9 g) wurde zusammen mit Biphenyl (0,45 g) und 1,4-Dicyano-2,3,5,6-tetramethylbenzol (0,22 g) in Acetonitril/Wasser (10:1) (330 mL) gelöst. Durch die Lösung wurde 15-20 min ein Argonstrom geleitet und 3 Tage im Flachbett-Sonnenlichtsammler belichtet (ca. 50 % Bewölkung und 50 % direkte Sonnenstrahlung), bis dünnschichtchromatographisch kein D mehr nachweisbar war. Anschließend wurde die Lösung im Rotationsverdampfer bis zur Trockne eingeengt und das feste, farblose bis gelbe Produktgemisch säulenchromatopraphisch (Kieselgel, n-Pentan/Diethylether/Ethylacetat 5:1:0 bis 30:15:1) getrennt (E, Ausbeute 15-25%).
**E** (1 g) wurde in abs. Methanol (40 mL) gelöst und unter Argon bei 10°C mit Natriummethanolat in abs. Methanol (25 mL 0,5molar) versetzt und bei Raumtemperatur gerührt. Zugabe von 80 mL Wasser und mit 300 mL Diethylether extrahiert. Säulechromatographische Reinigung an Kieselgel (Dichlormethan/Diethylether 1:1): 0,73 g F (95% Ausbeute).

Für analoge Beispiele von Umsetzungen mit kürzer-kettigen terpenoiden Polyalkenen (Geranyl- und Farnesylderivate), siehe M. Demuth, *Pure Appl. Chem.* **1999**, im Druck.

Analytische Daten zur Struktursicherung von **D,E** und **F**, siehe Doktorarbeit von C. Heinemann, MPI für Strahlenchemie/ Universität Essen (1998)

### Beispiel 7

Steroidsynthese (komplexes Gerüst)

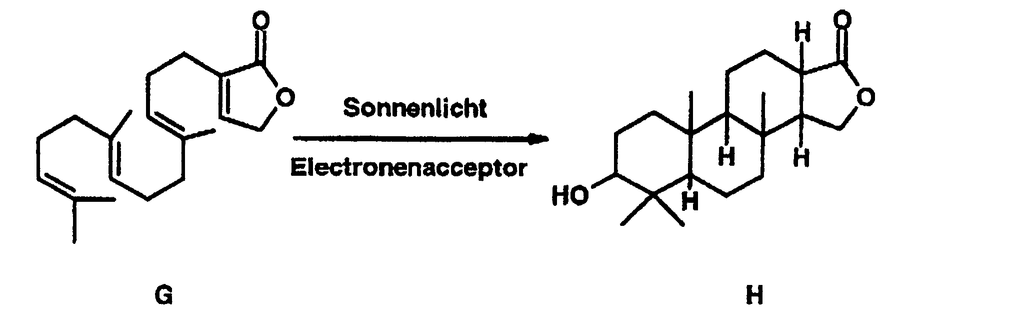

Eine Lösung bestehend aus 220 ml Acetonitril/ Wasser (10:1), 0,5 g 3-Farnesylmethyl-2(5H)-furanon (**G**), 0,094 g Trimethyl-dicyanobenzol und 0,269 g Biphenyl wurden im Flachbett-Sonnenlicht-Sammler mit 0,1 qm Fläche 5 Tage belichtet (sonnig, bewölkt und teilweise Regen).

Nach dem Einengen der Lösung wurde der Rückstand chromatographisch an Kieselgel 60 (0,04-0,063 mm, Merck, 100-fach) mit Pentan-Ether 2:1 gereinigt. Es wurden 0,0779 g 3-Hydroxy-spongian-16-on (**H**) erhalten (15% d.Th.).

Analytische Daten zur Struktursicherung von **G** und **H**, siehe laufende Doktorarbeit von F. Göller, MPI für Strahlenchemie/Universität Essen.

### Beispiel 8

Herstellung von Rosenoxid (**K**) mittels Sonnenlicht (Photooxigenierung von Citronellol). 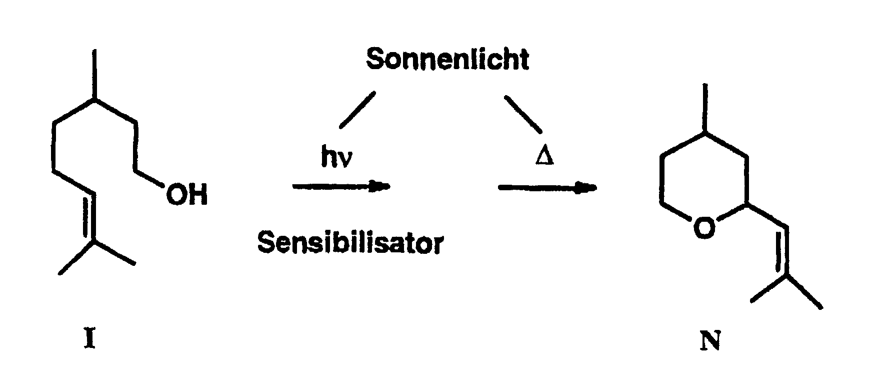
500 ml ( 429 g, 2.7 Mol Citronellol (**I**)
400 ml Methanol (**J**)
30 g Bengalrosa (**K**)

**I**, **J** und 6g von **K** werden zusammengegeben und im Flachbett-Sonnenlichtsammler unter Durchleiten von Luft belichtet. Wenn die dunkelrote Lösung wärend des Belichtens braun wird, so wird nochmals 6 g an K hinzugegeben. Dies wurde wärend des Belichtens mit Sonnenlicht über 3 Tage, (50 % Bewölkung und 50 % direkte Strahlung) viermal notwendig.

Der Reaktionsverlauf kann mittels Dünnschichtchromatographie verfolgt werden.

Zu der belichteten Lösung werden 800 ml konz. Na₂SO₃-Lösung (**L**) gegeben und für 3 Stunden im Flachbett-Sonnenlichtsammler auf 70 °C erwärmt, wobei dies auch durch direkte Sonnenstrahlung geschehen kann. Nach dem Abkühlen auf Raumtemperatur gibt man die Lösung in einen Scheidetrichter und trennt die untere (wäßrige) Phase ab.

Die im Scheidetrichter verbleibende Phase wird in einen Kolben gegeben, zu der 500 ml 5%-iger Schwefelsäure (**M**) gegeben wird. Nach 30 minütiger Reaktionszeit wird ein Wasserdampfstrom durchgeleitet und das übergetriebene Oxidgemisch in einer Vorlage aufgefangen. Nach Abtrennung des Wassers verbleiben 210 g Rosenoxid (**N**) zurück.

Analytische Daten zur Struktursicherung von **I** und **N**, siehe Patentschrift EP0842926 Dragoco Gerberding & Co AG

### Beispiel 9

Verwendung von in Reihe geschalteten Flachbettkollektoren:
Herstellung von Rosenoxid (**N**) mittels Sonnenlicht
Wie in allen vorangegangenen Beispielen wurden Reaktoren gleicher Gesamtfläche (1 m²) verwendet.

200 ml (172 g, 1.1 Mol Citronellol (**I**)
11 Methanol (**J**)
4 g Bengalrosa (**K**)

**I**, **J** und **K** (3 g) werden zusammengegeben und im Flachbett-Sonnenlichtsammler, bestehend aus zwei in Reihe geschalteten Flachbett-Sonnenlichtsammlern mit je 0.5 m² Reaktorfläche, unter Durchleiten von Luft belichtet. Die Reaktionslösung wurde dabei mit ca. 2 l/h durch die Reaktoren gepumpt und so eine fliessende/kontinuierliche Produktion ermöglicht. Wenn die dunkelrote Lösung wärend des Belichtens braun wird, so wird nochmals 1g an **K** hinzugegeben. Die Reaktionszeit betrug 11 Std. bei 70% Bewölkung und 30% Sonnenschein.

Zu der belichteten Lösung werden 300 ml konz. Na₂SO₃-Lösung (**L**) gegeben und für 3 Stunden im Flachbett-Sonnenlichtsammler auf 70 °C erwärmt, wobei dies auch durch direkte Sonnenstrahlung geschehen kann. Nach dem Abkühlen auf Raumtemperatur gibt man die Lösung in einen Scheidetrichter und trennt die untere (wäßrige) Phase ab und die im Scheidetrichter verbleibende Phase wird in einen Kolben gegeben und mit 200 ml 5%-iger Schwefelsäure (**M**) versetzt. Nach 30-minütiger Reaktionszeit wird ein Wasserdampfstrom durchgeleitet und das übergetriebene Oxidgemisch in einer Vorlage aufgefangen. Nach Abtrennung des Wassers verbleiben 88 g Rosenoxid (**N**) zurück.

Analytische Daten zur Struktursicherung von **1** und **N**, siehe Patentschrift EP0842926 Dragoco Gerberding & Co AG (1998)

### Beispiel 10

Herstellung eines stereochemische und strukturell komplexen Stoffes 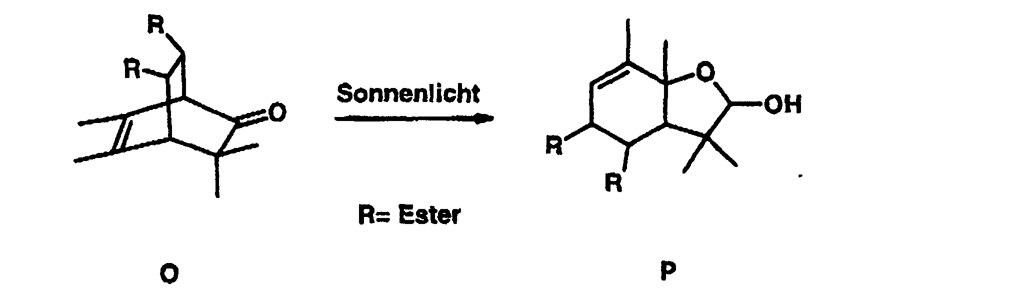 0,4 g 3,3,5,6-Tetramethyl-7,8-bismethoxycarbonylbicyclo[2.2.2]oct-5-en-2-on (**O**) wurden in 200 ml Acetonitril-Wasser (9:1) gelöst und im Flachbett-Sonnenlicht-Sammler (0,5 m² Reaktorfläche) 8 Stunden (Sonnenschein, bedeckt, regenerisch) unter Argon belichtet. Die Lösung wurde nach beendeter Belichtung eingeengt und an Kieselgel 60 (0,04-0,063 mm, Merck, 100-fach) mit Pentan-Aceton 10:1 gereinigt.
Es wurden 0,37 g an 9-Oxa-8-methoxy-1,2,7,7-tetramethyl-4,5-bismethoxycarbonylbicyclo-[4.3.0]non-2-en (**P**) erhalten (48% d.Th.).

Ein identischer Ansatz dauert im Parabolrinnenkonzentrator 3 Tage.
Analytische Daten zur Struktursicherung von **O** und **P**, siehe Doktorarbeit von A. Hülsdünker, MPI für Strahlenchemie/ Universität Essen (1994).

### Beispiel 11

Vergleich von in Reihe geschalteten Flachbettkollektoren mit Parabolinnenkollektor:
Photochemische Umlagerung (Oxa-di-pi-methan-Umlagerung) 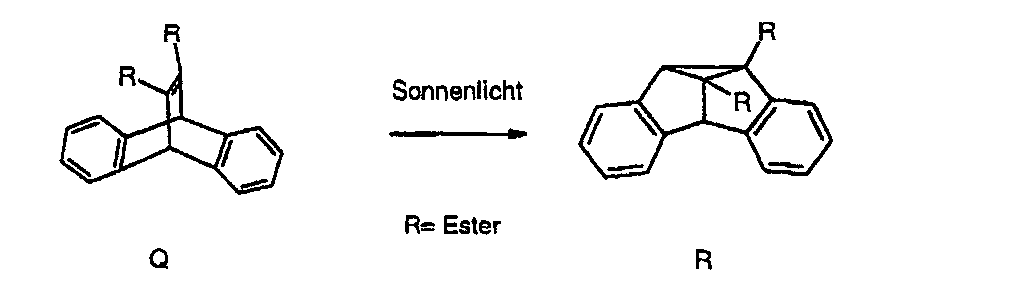 Eine Lösung, bestehend aus Acetonitril (1,9 1), 11 g 9,10-Dihyro-9,10-(11,12-bismethoxycarbonyl)ethenoanthracen (**Q**) und 75 ml Acetophenon, wurde je zur Hälfte in einem Parabolrinnenkollektor und in einem Flachbett- Sonnenlichtsammler belichtet.

Der Flachbett-Sonnenlichtsammler wurde wie in Beispiel 9 mit zwei in Reihe geschalteten Kollektoren mit je 0.5 m² Fläche betrieben, wobei die Lösung umgepumpt wurde.

### Aufarbeitung:

Nach dem Einengen der Lösung und Chromatographieren des Rückstandes an Kieselgel 60 (Merck, 80-fach) mit Pentan-Ether (90:10) wurden aus der Belichtung im Flachbett-Sonnenlichtsammler 4,45 g Produkt (R) (81% d.Th.) erhalten. Im Konzentrator entstanden nach gleicher Belichtungszeit (90 Min.) 0,9 g (ca. 16% d.Th.) Produkt (**R**).

Analytische Daten zur Struktursicherung von **Q** und **R**, siehe Doktorarbeit von A. Hülsdünker, MPI für Strahlenchemie/ Universität Essen (1994).

### Beispiel 12

Cycloaddition (Anlagerungsreaktion) als Vorstufe zur Herstellung des Taxan-ABC-Ringgerüstes (Vorstufe eines komplexen biologischen Wirkstoffs).

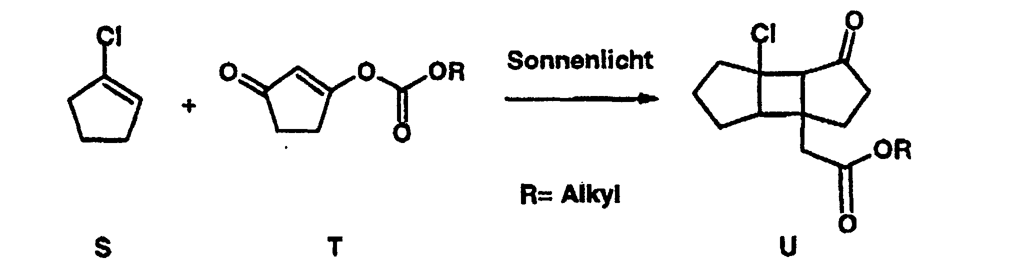

Eine Lösung von 18.5 g (**T**) in einem gemisch aus 180 ml (**S**) und 360 ml Toluol wurden im Flachbett-Sonnenlichtsammler (0,5 m² Reaktorfläche) 10 Stunden unter Argon belichtet. Nach dem Entfernen des Lösungsmittels wurde durch Säulenchromatographie (Kieselgel 60 (0,04-0,063 mm), mit dem Laufmittelmittelgemisch Pentan/Ethylacetat = 20 : 1 → 8 : 1 gereinigt und (**U**) in einer Ausbeute von 54% erhalten.
Ein identischer Ansatz dauert im Parabolrinnenkonzentrator 4 Tage.

Analytische Daten zur Struktursicherung von **S**,**T** und **U**, siehe Doktorarbeit von D. Sträubig, MPI für Strahlenchemie/ Universität Essen (1997).

## Patentansprüche

1. Verfahren zur Herstellung von Riech- und Aromastoffen, von Steroiden und deren Derivaten und von komplexen biologischen Wirkstoffen und deren Vorstufen durch Elektronenübertragungsreaktionen oder durch Umlagerungs- und Anlagerungsreaktionen mit Hilfe von solar-photo- und solar-thermochemischen Synthesen, **dadurch gekennzeichnet, dass** die Synthesen in einem Flachbett-Sonnenlichtsammler bei Einstrahlung von diffusem Sonnenlicht oder diffusem Kunstlicht ohne Vorrichtungen zur Fokussierung durchgeführt werden.

2. Verfahren nach Anspruch 1, wobei die Riech- und Aromastoffen durch Photooxigenierung hergestellt werden.

3. Verfahren nach Anspruch 2 zur Herstellung von Rosenoxid, Myrtenol oder Myrtenal.

4. Verfahren nach Anspruch 1, wobei die Innenseiten des Flachbett-Sonnenlichtsammlers mit strahlungsreflektierenden Oberflächen ausgestattet sind.

5. Verfahren nach Anspruch 1-4, wobei der Flachbett-Sonnenlichtsammler mit einer oder mehreren transparenten, wellenlängenselektiven Abdeckungen versehen ist.

6. Verfahren nach Anspruch 5, wobei als wellenlängenselektive Abdeckung eine Fluorpolymerfolie eingesetzt wird.

7. Verfahren nach Anspruch 1-6, wobei an den Flachbett-Sonnenlichtsammler ein Wärmetauscher angeschlossen ist, über den Reaktionswärme abgeführt wird.

8. Verfahren nach Anspruch 7, wobei der Wärmetauscher zur Kühlung des Flachbett-Sonnenlichtsammlers zusätzlich an einen Erdwärmetauscher angeschlossen ist.

9. Verfahren nach Anspruch 1-6, wobei an den Flachbett-Sonnenlichtsammler ein Wärmetauscher angeschlossen ist, an den ein Prozesswärme liefernder Solarkollektor oder Flachbett-Sonnenlichtsammler angeschlossen ist.

10. Verfahren nach Anspruch 1, wobei anstelle eines einzelnen Flachbett-Sonnenlichtsammlers mehrere miteinander im Durchfluss verbundene Flachbett-Sonnenlichtsammler eingesetzt werden.

11. Verfahren nach Anspruch 10, **dadurch gekennzeichnet, dass** mit Hilfe eines Pumpensystems das in der solar-photo- oder solar-thermochemischen Synthese hergestellte Produkt kontinuierlich aus dem letzten Flachbett-Sonnenlicht-Sammler (Kollektor) aus einer Reihe miteinander verbundener Kollektoren abgezogen wird und eine entsprechende Menge Ausgangsprodukt in den ersten Kollektor der Reihe nachgeführt wird.

## Claims

1. A process for the production of flavours/fragrants and aromas, of steroids and derivatives thereof, and of complex biologically active agents and precursors thereof by means of electron-transfer reactions or by means of rearrangements and addition reactions using solar-photochemical and solar-thermochemical syntheses, **characterized in that** the syntheses are performed in a flat-bed solar collector upon irradiation with diffuse sunlight or diffuse artificial light without devices for focusing.

2. The process of claim 1 wherein said flavours/fragrants and aromas are produced by means of photooxygenation.

3. The process of claim 2 for the production of rose oxide, myrtenol or myrtenal.

4. The process of claim 1 wherein the interior sides of the flat-bed solar light collector are provided with radiation-reflecting surfaces.

5. The process of claims 1-4 wherein the flat-bed solar light collector is equipped with one or more transparent, wavelength-selective covers.

6. The process of claim 5 wherein the wavelength-selective cover is a fluorinated polymer sheet.

7. The processes of claims 1-6 wherein the flat-bed solar light collector is coupled to a heat exchanger through which the heat of reaction is dissipated.

8. The process of claim 7 wherein the heat exchanger is additionally connected to an underground heat exchanger for cooling the flat-bed solar light collector.

9. The process of claims 1-6 wherein the flat-bed solar light collector is coupled to a heat exchanger which is coupled to a process heat delivering solar collector or flat-bed solar light collector.

10. The process of claim 1 wherein instead of a single flat-bed solar light collector several flat-bed solar light collectors are connected in series and are operated at continuous flow.

11. The process of claim 10, **characterized in that** the product which is produced by said solar-photochemical or solar-thermochemical synthesis is continuously collected and removed from the last flat-bed solar light collector in a series of interconnected collectors by means of a pumping system, and a corresponding amount of starting material is recharged into the first collector of the series.

## Revendications

1. Procédé de fabrication de matières odorantes et d'arômes, de stéroïdes et de leurs dérivés, ainsi que de substances biologiques complexes et de leurs précurseurs, par réactions de transfert d'électrons ou par réactions de transposition et de fixation à l'aide de synthèses photochimiques solaires et thermochimiques solaires, **caractérisé en ce que** les synthèses sont conduites dans un collecteur de lumière solaire à fond plat par irradiation de lumière solaire diffuse ou de lumière artificielle diffuse sans dispositifs de focalisation.

2. Procédé selon la revendication 1, dans lequel les matières odorantes et d'arômes sont fabriquées par photo-oxygénation.

3. Procédé selon la revendication 2 pour fabriquer de l'oxyde de rose, du myrténol ou du myrténal.

4. Procédé selon la revendication 1, dans lequel les faces internes du collecteur de lumière solaire à fond plat sont munies de surfaces réfléchissant le rayonnement.

5. Procédé selon les revendications 1 à 4, dans lequel le collecteur de lumière solaire à fond plat est équipé d'un ou de plusieurs revêtements transparents à sélection de longueurs d'onde.

6. Procédé selon la revendication 5, dans lequel on utilise comme revêtement à sélection de longueurs d'onde une feuille de fluoropolymère.

7. Procédé selon les revendications 1 à 6, dans lequel on raccorde au collecteur de lumière solaire à fond plat un échangeur de chaleur par lequel la chaleur de réaction est évacuée.

8. Procédé selon la revendication 7, dans lequel l'échangeur de chaleur, pour refroidir le collecteur de lumière solaire à fond plat, est en outre raccordé à un échangeur géothermique.

9. Procédé selon les revendications 1 à 6, dans lequel on raccorde au collecteur de lumière solaire à fond plat un échangeur de chaleur, auquel est raccordé un collecteur solaire fournissant la chaleur du processus ou un collecteur de lumière solaire à fond plat.

10. Procédé selon la revendication 1, dans lequel, à la place d'un collecteur individuel de lumière solaire à fond plat, on utilise plusieurs collecteurs de lumière solaire à fond plat reliés ensemble en série.

11. Procédé selon la revendication 10, **caractérisé en ce que**, à l'aide d'un système à pompe, le produit, fabriqué lors de la synthèse photochimique solaire ou thermochimique solaire, est soutiré en continu du dernier collecteur de lumière solaire à fond plat (collecteur) d'une série de collecteurs reliés entre eux, et **en ce que** l'on amène dans le premier. collecteur de la série une quantité correspondante de produit initial.
